(19) Europäisches Patentamt
European Patent Office
Office européen des brevets

(11) Publication number: 0 232 025 A2

# (12) EUROPEAN PATENT APPLICATION

(21) Application number: 87300328.9

(22) Date of filing: 15.01.87

(51) Int. Cl.4: C 07 D 401/12
C 07 D 211/90,
C 07 D 417/12,
C 07 D 405/12, A 61 K 31/44

(30) Priority: 21.01.86 GB 8601382

(43) Date of publication of application:
12.08.87 Bulletin 87/33

(84) Designated Contracting States:
AT BE CH DE ES FR GB GR IT LI LU NL SE

(71) Applicant: Pfizer Limited
Ramsgate Road
Sandwich Kent CT13 9NJ (GB)

(72) Inventor: Alker, David Dr.
20, Hunting Gate
Birchington Kent (GB)

Campbell, Simon Fraser, Dr.
Grey Friars Upper Street
Kingsdown Deal Kent (GB)

Cross, Peter Edward, Dr.
21, Cherry Avenue
Canterbury Kent (GB)

(74) Representative: Moore, James William, Dr.
Pfizer Limited Ramsgate Road
Sandwich Kent CT13 9NJ (GB)

## (54) Dihydropyridine anti-ischaemic and antihypertensive agents.

(57) 1.4-Dihydropyridine derivatives of the formula:-

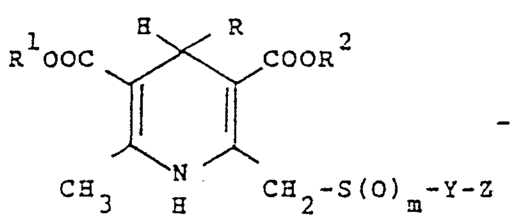

--- (I)

wherein R is an aryl or heteroaryl group; $R^1$ and $R^2$ are each independently $C_1$-$C_4$ alkyl optionally substituted by $C_3$-$C_7$ cycloalkyl, aryl, $CF_3$, $C_2$-$C_4$ alkanoyl, halo, hydroxy, cyano, $C_1$-$C_4$ alkoxy, $C_2$-$C_4$ alkanoyloxy or $NR^3R^4$ where $R^3$ and $R^4$ are each independently H, $C_1$-$C_4$ alkyl, aryl-($C_1$-$C_4$ alkyl) or together with the nitrogen atom to which they are attached represent a pyrrolidinyl piperidino, morpholino, piperazinyl, 4-($C_1$-$C_4$ alkyl)piperazin-1-yl or 4-formylpiperazin-1-yl group; m is 0, 1 or 2; Y is an optional alkylene linking group of from 0 to 4 carbon atoms which may be branched; and Z is Het, -O-Het or -NH-Het where Het is an optionally substituted heterocyclic group, are calcium channel blockers useful as anti-ischaemic and antihypertensive agents.

EP 0 232 025 A2

## Description

"Dihydropyridine Anti-ischaemic and Antihypertensive Agents"

The invention relates to certain dihydropyridines. specifically to certain 1,4-dihydropyridines having a heterocyclic substituent in a thioether side chain attached to the 2-position. and derivatives thereof. which have utility as anti-ischaemic and antihypertensive agents.

The compounds of the invention reduce the movement of calcium into the cell and they are thus able to delay or prevent the cardiac contracture which is believed to be caused by an accumulation of intracellular calcium under ischaemic conditions. Excessive calcium influx during ischaemia can have a number of additional adverse effects which would further compromise the ischaemic myocardium. These include less efficient use of oxygen for ATP production. activation of mitochondrial fatty acid oxidation and possibly. promotion of cell necrosis. Thus the compounds are useful in the treatment or prevention of a variety of cardiac conditions. such as angina pectoris. cardiac arrhythmias. heart attacks and cardiac hypertrophy. The compounds also have vasodilator activity since they can inhibit calcium influx in cells of vascular tissue and they are thus also useful as antihypertensive agents and for the treatment of coronary vasospasm.

In addition, because of their ability to inhibit calcium influx into smooth muscle cells, it is expected that they will have activity in the treatment of congestive heart failure, gastrointestinal disorders, cerebrovascular disorders, peripheral vascular disease, bronchopulmonary disorders, pulmonary hypertension, smooth muscle disorders of the urino-genital system, and hypertrophic cardiac myopathy.

A number of 4-aryl-1,4-dihydropyridine calcium antagonists are known, including for example the agent nifedipine. The class has been reviewed by Bossert et. al., Angew. Chem. Int. Ed., 1981, 20, 762. Our European patent applications publication nos. 100189, 106462, 107293, 116769, 132375 and 150939 disclose various dihydropyridines having a substituted alkoxyalkyl group at the 2-position.

According to the present invention, there are provided novel 1,4-dihydropyridine derivatives of the formula:-

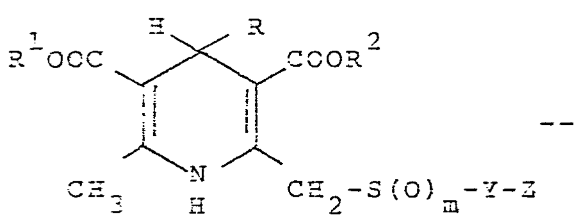

--- (I)

and their pharmaceutically acceptable salts,
where R is an aryl or heteroaryl group;
$R^1$ and $R^2$ are each independently $C_1$-$C_4$ alkyl optionally substituted by $C_3$-$C_7$ cycloalkyl, aryl, $CF_3$, $C_2$-$C_4$ alkanoyl, halo, hydroxy, cyano, $C_1$-$C_4$ alkoxy, $C_2$-$C_4$ alkanoyloxy or $NR^3R^4$ where $R^3$ and $R^4$ are each independently H, $C_1$-$C_4$ alkyl, aryl-($C_1$-$C_4$ alkyl) or together with the nitrogen atom to which they are attached represent a pyrrolidinyl, piperidino, morpholino, piperazinyl, 4-($C_1$-$C_4$ alkyl)piperazin-1-yl or 4-formylpiperazin-1-yl group;
m is 0, 1 or 2;
Y is an optional alkylene linking group of from 0 to 4 carbon atoms which may be branched;
and Z is Het, -0-Het or -NH-Het where Het is an optionally substituted heterocyclic group.

The term "aryl" as used in this specification includes unsubstituted phenyl and phenyl substituted by, for example, one or two substituents each independently selected from nitro, halo. $C_1$-$C_4$ alkyl, $C_1$-$C_4$ alkoxy, hydroxy. trifluoromethyl and cyano. It also includes 1- and 2-naphthyl.

The term "heteroaryl" as used in this specification for R means an aromatic heterocyclic group which may optionally be substituted and includes, for example, benzofuranyl; benzothienyl; pyridyl optionally substituted by methyl, methylthio. halo or cyano; quinolyl; benzoxazolyl; benzothiazolyl; furyl; pyrimidinyl; thiazolyl; 2,1,3-benzoxadiazol-4-yl; 2,1,3-benzothiadiazol-4-yl; and thienyl optionally monosubstituted by halo or $C_1$-$C_4$ alkyl.

Alkyl and alkoxy groups having 3 or more carbon atoms can be straight or branched chain.

"Halo" means F, Cl, Br or I.

R is preferably phenyl substituted by 1 or 2 substituents each selected from halo and $CF_3$ or is 2-chloropyrid-3-yl. Particularly preferred examples of R are 2-chlorophenyl and 2,3-dichlorophenyl.

$R^1$ and $R^2$ are preferably $CH_3$ or $C_2H_5$, the particular cases where $R^1$ is $CH_3$ and $R^2$ is $C_2H_5$, or $R^1$ and $R^2$ are both $CH_3$ being especially preferred.

The group "Het" is a 5 or 6 membered heterocyclic group which may contain one or more N, S or O heteroatoms and which may be saturated, unsaturated or partially unsaturated and which may optionally be fused to a further 5 or 6 membered heterocyclic or carbocyclic ring and which may optionally be substituted.

When substituted, Het may be substituted where appropriate by up to three substituents which include such substituents as $C_1$-$C_4$ alkyl, $C_1$-$C_4$ alkoxy. $C_2$-$C_5$ alkanoyl, halo, oxo, CN, $-(CH_2)_nCO_2H$, $-(CH_2)_nCO_2(C_1$-$C_4$ alkyl), $-(CH_2)NR^3R^4$.n$-(CH_2)_nCONR^3R^4$, $-(CH_2)_nNHCONR^3R^4$ $-(CH_2)_nNHCOCF_3$,

$-(CH_2)_nNHCO(C_1-C_4$ alkyl), $-(CH_2)_nNHSO_2(C_1-C_4$ alkyl), $-(CH_2)_nNHSO_2$ aryl, $-(CH_2)_nNHSO_2NR^3R^4$, $-(CH_2)_nNHSO_2Het^1$, $-(CH_2)_nHet^1$, $-(CH_2)_nNHHet^1$, $-NH(CH_2)_nHet^1$, $-O-(CH_2)_nHet^1$, $-(CH_2)_nOH$, $-(CH_2)_n$aryl, -Oaryl, $-NH(CH_2)_pNR^3R^4$, $-C(C{=}X)NHR^5$, $-C({=}NCN)R^6$, $-C({=}NSO_2R^7)NH(C_1-C_4$ alkyl), $-C({=}NH)NHR^8$, $-C({=}NCO(C_1-C_4$ alkyl))$NHCO(C_1-C_4$ alkyl) and $-C({=}NR^9)SCH_3$;
wherein n is 0, 1, 2, 3 or 4; p is 2, 3 or 4;
$R^3$ and $R^4$ are as previously defined;
$R^5$ is H, $C_1-C_4$ alkyl, $C_3-C_6$ cycloalkyl, $-CO_2(C_1-C_4$ alkyl), $-CH_2CO_2(C_1-C_4$ alkyl), aryl, $-SO_2$aryl or $Het^1$;
X is O or S;
$R^6$ is $NR^3R^4$ or $-NH(CH_2)_2N(C_1-C_4$ alkyl$)_2$;
$R^7$ is $C_1-C_4$ alkyl or aryl;
$R^8$ is $-CONH(C_1-C_4$ alkyl) or $-CO_2$ $(C_1-C_4$ alkyl);
$R^9$ is CN, $-SO_2(C_1-C_4$ alkyl) or $-SO_2$aryl;
and $Het^1$ is a 5 or 6 membered nitrogen, oxygen, or sulphur containing heterocyclic group which may be saturated or unsaturated and which may optional include a further one or two nitrogen atoms in the ring and which may optionally be benzofused or substituted with for example, halo, $C_1-C_4$ alkyl, $C_1-C_4$ alkoxy, trifluoroacetyl, hydroxy, oxo, acetamido, arylamino, $CONR^3R^4$, $NR^3R^4$ or $SO_2NR^3R^4$ groups where $R^3$ and $R^4$ are as previously defined.

Typical groups represented by Het include pyrrolyl, pyrrolinyl, furyl, thienyl, imidazolyl, imidazolinyl, pyrazolyl, thiazolyl, thiazolinyl, triazolyl, triazinyl, thiadiazolyl, oxadiazolyl, tetrazolyl, pyridyl, pyrazinyl, pyrazolinyl, pyrimidinyl or a partially saturated derivative thereof, piperidyl, piperazinyl, purinyl, quinazolinyl, benzothiazolyl and quinoxalinyl, all of which may be substituted where appropriate with up to three substituents selected from those defined above.

When Z is Het, the group Het may be linked either by a ring carbon atom or, when present, a ring nitrogen atom, with the proviso that if Het is linked by a ring nitrogen atom, Y must have at least two carbon atom between Het and S. When Z is -O-Het or -NH-Het, Het must of course be attached to the adjacent heteroatom by a ring carbon atom and Y must contain at least two carbon atoms between S and the heteroatom.

In one preferred aspect of this invention the group Y is $CH_2$ of $(CH_2)_2$, Z is Het and Het is preferably pyridyl, pyrimidinyl, pyrazolyl, furyl, imidazolyl, triazolyl, tetrazolyl, thiazolyl, thiadiazolyl or oxadiazolyl. The heterocyclic ring may be substituted or unsubstituted. Preferred substituents are $C_1-C_4$ alkyl, particularly methyl, $C_1-C_4$ alkoxy, particularly methoxy, carbamoyl, carbamoylmethyl, oxo, hydroxy, hydroxyethyl, amino, methylamino, and dimethylamino groups. Thus, in this aspect particular and preferred examples of Z include 2,4,5-trimethyl-1-imidazolyl; 1-methyl-2-imidazolyl; 1,2,4-triazol-4-yl; 1,2,3-(1H)-triazol-1-yl; 4-carbamoyl--1,2,3-(1H)-triazol-1-yl; 3-amino-1,2,4-triazol-5-yl; 5-methyl- and 5-amino-1,3,4- thiadiazol-2-yl; 5-methyl-1,3,4-oxadiazol-2-yl; 1- and 2-methyl, 1- and 2-hydroxyethyl and 1- and 2-(carbamoylmethyl)tetrazol-5-yl; 2-amino-4- imidazolon-1-yl; imidazolidine-2,4-dion-1-yl; 4-amino-2- imidazolon-1-yl; 2-amino-4-pyrimidon-6-yl; 2,3-dimethyl-4-pryimidon-6-yl; 4-methoxy-2-methylpyrimidin-6-yl; 2-amino-4-methoxypyrimidin-6-yl, 2-methoxy-4-pyrimidon-6-yl, and 2,4-dimethoxypyrimidin-6-yl.

In a second preferred aspect of the invention of the group Y is absent, Z is Het and Het is linked directly to S by a ring carbon atom thereof and is preferably pyridyl, pyrimidinyl, pyrazolyl, imidazolyl, triazolyl, tetrazolyl, thiazolyl, thiadiazolyl or oxadiazolyl. The heterocyclic ring may be substitued or unsubstituted. Preferred substituents are $C_1-C_4$ alkyl, particularly methyl, $C_1-C_4$ alkoxy, particularly methoxy, carbamoyl, carbamoylmethyl, oxo, hydroxy, amino, methylamino, dimethylamino, hydroxyethyl or morpholinomethyl. Thus, in this aspect, particular and preferred examples of Z include 2-pyridyl, 2-furyl, 2-thiazolyl, 2-pyrimidinyl, 1,2,4-(1H)-triazol-3-yl, 5-methylamino-thiadiazol-2-yl, 5-morpholinomethyl-thiazol-2-yl, 1-hydroxyethyl-tetrazol-5-yl and 5-hydroxyethyl-thiadiazol-2-yl.

In a third preferred aspect of the invention, the group Y is preferably $CH_2$ or $(CH_2)_2$, Z is Het and Het is preferably a tetrazolyl, thiadiazolyl, thiazolyl, thienyl or furyl group substituted by a substituent selected from $-(CH_2)_nNR^3R^4$, $-(CH_2)_nNHCOCF_3$, $-(CH_2)_nNHCONR^3R^4$, $-(CH_2)_nNHSO_2(C_1-C_4$ alkyl), $-(CH_2)_nNH-Het^1$ and $-(CH_2)_nHet^1$ wherein n is 1, 2 or 3, $R^3$ and $R^4$ are as previously defined and $Het^1$ is a pyridyl, pyrimidinyl, triazinyl, imidazolyl, triazolyl, tetrazolyl, thiazolyl, thiadiazolyl or oxadiazolyl group and said $Het^1$ group is unsubstituted or substituted with $C_1-C_4$ alkyl, hydroxy, oxo, trifluoroacetyl, arylamino, carbamoyl, methylcarbamoyl, amino or di$(C_1-C_4$ alkyl)amino groups.

Thus, in this aspect, particular and preferred examples of Z include 5-tetrazolyl, 2-thiazolyl and 2-thiadiazolyl each of which is substituted by a substituent chosen from 2-pyridylmethyl, 4-imidazolylmethyl, 2-(4-morpholino)ethyl, 2-(1-piperazinyl)ethyl, 2-(4-trifluoroacetyl-1-piperazinyl)ethyl, 2-(4-methylcarbamoyl-1-piperazinyl)ethyl, 2-aminoethyl, 2-methylaminoethyl and 2-dimethylaminoethyl.

In a fourth preferred aspect of the invention Y is $CH_2$, Z is Het, and Het is a group of the formula:

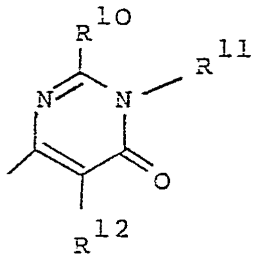

or

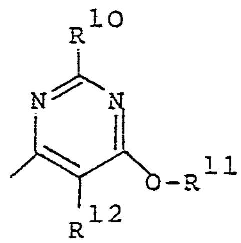

wherein $R^{10}$ is H, $Het^1$, $-(CH_2)_n-Het^1$, $-NH-Het^1$, $-NH-(CH_2)_n-Het^1$, $-O-Het^1$, $-O-(CH_2)_n-Het^1$, $-(CH_2)_n-OH$, $-NR^3OR^4$, $-(CH_2)_nNR^3R^4$ or $-NH-(CH_2)_pNR^3OR^4$; $R^{11}$ is H, $C_1$-$C_4$ alkyl, $-(CH_2)_pNR^3R^4$, $-(CH_2)_p-OH$ or $-(CH_2)_n-Het^1$; and $R^{12}$ is H, $C_1$-$C_4$ alkyl or phenyl; wherein $R^3$, $R^4$, $Het^1$ and p are as previously defined and n is 1, 2, 3 or 4.

In this aspect of the invention preferred substituents for $R^{10}$ include amino, aminomethyl, hydroxymethyl, 2-pyridyl, 4-morpholinomethyl, 2-pyridylmethylamino, or 4-pyridylmethyl-amino; preferred substituents for $R^{11}$ include 2-dimethylamino-ethyl, (1-methyl-2-imidazolyl)methyl, 2-hydroxyethyl and 2-pyridylmethyl. $R^{12}$ is preferably H. Thus in this aspect, particular and preferred groups for Z are 2-(2-pyridylmethyl-amino)pyrimid-4-on-6-yl, 2-amino-3-(2-pyridylmethyl)pyrimid-4-on-6-yl, 2-amino-3-[(1-methyl-2-imidazolyl)methyl]pyrimid-4-on-6-yl and 2-amino-4-(2-pyridylmethoxy)pyrimidin-6-yl.

In a fifth preferred aspect of the invention Y is $(CH_2)_2$, Z is NH-Het and Het is preferably a heterocyclic group selected from triazolyl, oxadiazolyl, pyrimidinyl or a partially saturated derivative thereof, purinyl, quinazolinyl, imidazolyl, imidazolinyl, triazinyl, pyridyl, thiazolyl, thiazolinyl, benzothiazolyl, thiadiazolyl, pyrazinyl and quinoxalinyl and their N- and S-oxides, Het being optionally substituted by $C_1$-$C_4$ alkyl, $C_1$-$C_4$ alkoxy, halo, hydroxy, oxo, cyano, 3-methylureido, phenyl, phenoxy, pyridyl, acetyl, carbamoyl, methylcarbamoyl, ($C_1$-$C_4$ alkoxy)carbonyl, $-NR^3R^4$, or $-SO_2NR^3R^4$ where $R^3$ and $R^4$ are as previously defined. In this aspect Het is most preferably 1,2,4-triazol-3-yl, 1,2,4-oxadiazol-3-yl, 1,2,5-thiadiazol-3-yl, pyrimidin-2-yl, pyrimidin-4-yl, imidazol-2-yl, 1,3,5-triazin-2-yl, pyrid-2-yl, thiazol-2-yl or pyrazin-2-yl optionally substituted as above, and thus particular and preferred examples of Z include 2-pyrazinyl, 2-pyrimidinyl, 6-(4-morpholino)pyrimidin-4-yl and 5-amino-1,2,4-triazol-3-yl.

In a sixth preferred aspect of the invention Y is $(CH_2)_2$, Z is O-Het and Het is preferably a heterocyclic group selected from triazolyl, pyrimidinyl, imidazolyl, pyrazolyl, pyridyl, thiazolyl and pyrazinyl, all optionally substituted by $C_1$-$C_4$ alkyl, $C_1$-$C_4$ alkoxy, halo, hydroxy, oxo, CN, phenyl, phenoxy, acetyl, ($C_1$-$C_4$ alkoxy)carbonyl, $NR^3R^4$ or $CONR^3R^4$ wherein $R^3$ and $R^4$ are as previously defined, (2-aminoethyl)amino, $(CH_2)_pOH$ where p is as previously defined, $-CH_2CH(OH)CH_2OH$, $-CH_2Het^1$ or $-OCH_2Het^1$ where $Het^1$ is preferably 2-pyridyl or 1-methylimidazol-2-yl. Thus, in this aspect of the invention particular examples of Het include 1,2,4-triazol-3-yl, pyrimidin-2-yl, pyrimidin-4-yl, imidazol-2-yl, pyrid-2-yl, thiazol-2-yl, pyrazin-2-yl and pyrazol-3-yl, optionally substituted as above.

In this aspect Het is most preferably a 2- or 4-pyrimidinyl group optionally substituted by carbamoyl, amino, halo, $C_1$-$C_4$ alkoxy, (2-aminoethyl)amino, (2-hydroxyethyl)amino, 2-pyridylmethylamino, (2,3-dihydroxypropyl)amino, 2-pyridylmethoxy, 1-methyl-2-imidazolylmethoxy or morpholino.

In a seventh preferred aspect of the invention Y is $(CH_2)_2$, Z is Het and Het is a group of the formula:

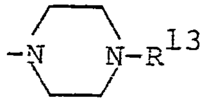

wherein $R^{13}$ is hydrogen or a group selected from $-C(=X)NHR^5$, $-CON(C_1$-$C_4$ alkyl$)_2$, $-C(=NCN)R^6$, $-C(=NSO_2R^7)NH(C_1$-$C_4$ alkyl), $-C(=NH)NHR^8$, $-C(=NCO(C_1$-$C_4$ alkyl)NHCO($C_1$-$C_4$ alkyl), $-C(=NR^9)SCH_3$, $-CH_2CONH_2$, $-CH_2CO_2(C_1$-$C_4$ alkyl), $-SO_2NH_2$, $-SO_2N(C_1$-$C_4$ alkyl$)_2$, $-SO_2$aryl, $-SO_2(C_1$-$C_4$ alkyl), $-CO_2(C_1$-$C_4$ alkyl), $-CO_2(C_1$-$C_4$ alkyl), aryl, or $Het^1$ wherein X, $R^5$, $R^6$, $R^7$, $R^8$ and $R^9$ are as previously defined and $Het^1$ is preferably pyrrolin-2-yl, imidazolin-2-yl, pyrimidin-4-on-2-yl, 5-amino-1,2,4-triazol-3-yl, thiazol-2-yl or 1,3,4-thiazol-2-yl.

As will be understood by those skilled in the art, hydroxyor oxo-substituents, if present in Het, can be subject to keto-enol tautomerism and the group may be present either as a free hydroxyl group when in the tautomeric enol form, or as an oxo group when in the keto form. Which particular isomer is present in normal circumstances can be readily determined by appropriate physical measurements, e.g. by infra-red spectroscopy, and, in some instances, the compound may exist as a mixture of the two forms. Both tautomers are of course within the scope of this invention.

The compounds of the formula (I) containing one or more asymmetric centres will exist as one or more pairs of enantiomers, and such pairs or individual isomers may be separable by physical methods, e.g. by fractional

crystallisation or chromatography of the parent compounds or of a suitable salt or derivatives thereof as will be known to those skilled in the art. The invention includes the separated pairs as well as racemic mixtures thereof and the separated optically-active isomeric forms.

The pharmaceutically acceptable acid addition salts of the compounds of the formula (I) which form such salts are those formed from acids which form non-toxic acid addition salts, for example the hydrochloride, hydrobromide, sulphate or bisulphate, phosphate or acid phosphate, acetate, citrate, fumarate, gluconate, lactate, maleate, succinate and tartrate salts. For some compounds, salts may also be formed with bases, examples are the sodium, potassium and ammonium salts.

The compounds of formula (I) may be prepared by the following methods.

1. The compounds of the invention in which Z is Het and m is 0 can be prepared by reacting a compound of the formula:

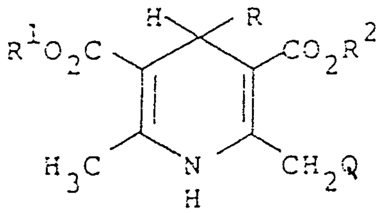

--- (II)

wherein R, $R^1$ and $R^2$ are as previously defined and Q is a good leaving group, with a thiol of the formula:

HS-Y-Het ---(III)

wherein Y and Het are as previously defined.

The leaving group Q may be for example a halo atom, preferably bromo, or a para-toluenesulphonyloxy, methanesulphonloxy, trifluoromethanesulphonyloxy or 1-imidazolylsulphonyloxy group. The reaction with the thiol of formula (III) is generally achieved by stirring the reactants in more or less equimolar proportions in an inert organic solvent e.g. tetrahydrofuran or N,N-dimethylformamide, in the presence of a base, e.g. potassium carbonate. The reaction is generally complete within a few hours at room temperature and the desired product is then isolated and purified using conventional techniques.

The compounds of the formula (I) in which m is 1 or 2, (i.e. the sulphinyl and sulphonyl derivatives) can be prepared by the oxidation of the corresponding thioether compounds, typically by using about one equivalent of a suitable oxidising agent such as meta-chloroperbenzoic acid or sodium metaperiodate to give the sulphinyl compounds or two or more equivalents to give the sulphonyl derivatives.

The starting materials of formula (II) are generally known compounds described in the literature or obtainable following literature precedents. Thus for example, in one process the corresponding 2-methyl-1,4-dihydropyridine may be halogenated to yield the corresponding compound of formula (II) wherein Q is halo. The reaction may for example be performed using pyridinium bromide perbromide to yield the 2-bromomethyl derivative.

In an alternative process the corresponding 2-hydroxymethyl-1,4-dihydropyridine may be converted to, for example, the 2-halomethyl, 2-para-toluenesulphonyloxymethyl. 2-methanesulphonyloxymethyl or 2-trifluoromethylsulphonyoxymethyl derivative of formula (II) by conventional methods. Thus, for example, in one particular variant of this process, reaction of the 2-hydroxymethyl-1,4-dihydropyridine with sulphuryl chloride in N,N-dimethylformamide followed by addition of imidazole initially yields the compound of formula (II) wherein Q is a 1-imidazolylsulphonyloxy group. This compound in fact breaks down by reaction with the dihydropyridine nitrogen atom to yield a cyclic sulphamate ester which is reacted with the thiol of formula (III).

The thiols of formula (III) are generally known compounds which are either commercially available or they may be prepared by conventional reaction in accordance with literature precedents.

In some instances specific reactions can be used to form particular heterocyclic groups, for example by cyclisation of an appropriate precursor. Thus, for example, compounds of the formula (I) wherein Z is a tetrazolyl group may be prepared by reaction of the appropriate 2-[(1,4-dihydropyrid-2-yl)methylthio]-acetonitrile with tri-n-butyltin azide. The reaction is readily performed by warming the reactants together, generally in equimolar amounts, in an organic solvent e.g. dioxan. After a period of from 12 to 24 hours under reflux the solvent is evaporated and the resulting tin complex decomposed with acid. The desired product may then be isolated and purified by conventional techniques.

Additionally, when Het is substituted, the substituent may be indtroduced in a subsequent reaction or by a conventional chemical transformation reaction by converting one substituent into another. For example, a conventional alkylation reaction using a haloalkane or substituted-haloalkane may be used to introduce an alkyl or substituted-alkyl substituent. Thus, for example, reaction of a compound of formula (I) wherein Z is a 5-tetrazolyl group with 2-bromoethanol by refluxing in acetonitrile in the presence of a base yields the (2-hydroxyethyl)tetrazolyl derivative as a mixture of the 1- and 2-substituted isomers which may be separated by conventional chromatographic techniques.

Other examples which may be prepared by specific reactions include the compounds of formula (I) wherein Z is 2.4,5-trimethylimidazol-1-yl, which may be prepared by reaction of the appropriate 2-(2-aminoalkyl)thiomethyl-1,4-dihydropyridine with acetaldehyde ammonia and diacetyl; the compounds of formula (I) wherein Z

is 2-amino-4-pyrimidon-6-yl, which may be prepared by reaction of the appropriate [(1,4-dihydropyrid-2-yl)methylthio-alkyl]acetoacetate with guanidine; and the compounds of formula (I) wherein Z is 1,2,4-triazol-4-yl which may be prepared by reaction of the appropriate 2-(2-aminoalkyl)-thiomethyl-1,4-dihydropyridine with N,N-dimethylformamide azine.

Examples of these and other similar reactions are to be found in our European patent applications previously referred to.

2. The compounds of the invention in which Z is -NH-Het and m is 0 can be prepared by reacting a compound of the formula:

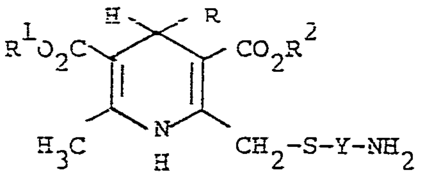

--- (IV)

wherein R, $R^1$ and $R^2$ are as previously defined and Y is an alkylene linking group of from 2 to 4 carbon atoms having at least 2 carbon atoms between S and $NH_2$; with a compound of the formula:

Het-Q --- (V)

wherein Q is a good leaving group. Examples of suitable leaving groups for Q in this case are $-NHNO_2$, $C_1$-$C_4$ alkylthio, $C_1$-$C_4$ alkoxy and halo.

The reaction is generally achieved by treating the reactants in more or less equimolar amounts in an organic solvent. Thus, for example, reaction of the compound of formula (IV) with 2-chloropyrimidine by refluxing in ethanol for 12 to 24 hours in the presence of triethylamine yields the compound of formula (I) wherein Z is (2-pyrimidinyl)amino.

As before, certain substituents can be introduced into the Het group by using conventional chemical transformations. Thus use of 4,6-dichloropyrimidine in the reaction with the compound of formula IV yields the 4-chloropyrimidin-6-yl derivative which may be reacted with an amine, e.g. morpholine to give the compound of formula (I) wherein Z is (4-morpholinopyrimidin-6-yl)amino.

Other examples together with further variations and possibilities will be evident to those skilled in the art and further examples are to be found in our European patent application no. 116769.

Compounds where m is 1 or 2 can be prepared by oxidation of the compounds where m is 0 as previously described.

3. The compounds of the invention in which Z is -0-Het and m is 0 can be prepared by reacting a compound of the formula:

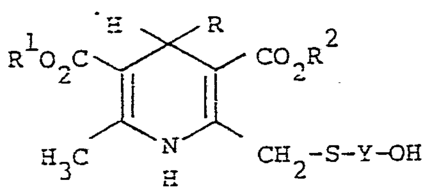

--- (VI)

wherein R, $R^1$ and $R^2$ are as previously defined and Y is an alkylene linking group of from 2 to 4 carbon atoms having at least 2 carbon atoms between S and O; with a strong base to form the anion, followed by addition of a compound of the formula (V).

The reaction is typically carried out by adding a strong base such as sodium hydride or potassium t-butoxide to a solution of the dihydropyridine (VI) in a suitable organic solvent (e.g. tetrahydrofuran) followed by stirring for a short period of time so as to form the base salt of the compound of formula (VI). The compound of formula (V) is then added. The reaction usually proceeds at room temperature but, if necessary, the reaction mixture can be heated to increase the rate of reaction. The compound of formula (I) is isolated and purified if necessary by conventional techiques.

As before conventional chemical transformation reactions can be used to introduce substituents into Het or to convert one substituent into another, and oxidation reactions yield the compounds wherein m is 1 or 2.

The ability of the compounds to inhibit the movement of calcium into the cell is shown by their effectiveness in reducing the contraction of vascular tissue in vitro which is the consequence of calcium influx caused by a high extracellular concentration of potassium ions. The test is performed by mounting spirally cut strips of rat aorta with one end fixed and the other attached to a force transducer. The tissue is immersed in a bath of physiological saline solution containing calcium ions at a concentration of 2.5 millimolar and potassium ions at a concentration of 5.9 millimolar. Potassium chloride solution is added to the bath with a pipette to give a final potassium ion concentration of 45 millimolar. The change in tension caused by the resulting contraction of the tissue is noted. The bath is drained and refilled with fresh saline solution containing the particular compound under test. After 45 minutes, the procedure is repeated and the contraction again noted. The concentration of

compound required to reduce the response by 50% is determined.

The antihypertensive activity of the compounds is evaluated after oral administration by measuring the fall in blood pressure in spontaneously hypertensive rats or renally hypertensive dogs.

For administration to man in the curative or prophylactic treatment of cardiac conditions and hypertension, oral dosages of the compounds will generally be in the range of from 2-100 mg daily for an average adult patient (70 kg). Thus for a typical adult patient, individual tablets or capsules contain from 1 to 20 mg of active compound, in a suitable pharmaceutically acceptable vehicle or carrier. Dosages for intravenous administration would typically be within the range 1 to 10 mg per single dose as required. In practice the physician will determine the actual dosage which will be most suitable for an individual patient and it will vary with the age, weight and response of the particular patient. The above dosages are exemplary of the average case but there can, of course, be individual instances where higher or lower dosage ranges are merited, and such are within the scope of this invention.

For human use, the compounds of the formula (I) can be administered alone, but will generally be administered in admixture with a pharmaceutical carrier selected with regard to the intended route of adminstration and standard pharmaceutical practice. For example, they may be administered orally in the form of tablets containing such excipients as starch or lactose, or in capsules or ovules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic with blood.

Thus in a further aspect the invention provides a pharmaceutical composition comprising a compound of the formula (I), or a pharmaceutically acceptable salt thereof, together with a pharmaceutically acceptable diluent or carrier.

The invention also includes a compound of the formula (I), or a pharmaceutically acceptable salt thereof, for use in medicine, in particular in the treatment of ischaemic heart disease, angina or hypertension in a human being.

The preparation of the compounds of the invention is illustrated by the following Examples:

## EXAMPLE 1

3,5-Bis(methoxycarbonyl)-4-(2-chlorophenyl)-6-methyl-2-(2-pyrimidinylthio)methyl-1,4-dihydropyridine

Pyridinium bromide perbromide (2.00 g) was added in one portion to a stirred, ice-cooled solution of 3,5-bis(methoxycarbonyl)-4-(2-chlorophenyl)-2,6-dimethyl-1,4-dihydropyridine (1.68 g) and pyridine (0.80 ml) in dichloromethane (15 ml). The mixture was stirred at 0°C for one hour and evaporated without external heating. The residue was partitioned between ethyl acetate and 2M hydrochloric acid. The layers were separated and the organic layer washed with iced-water, dried over sodium sulphate and evaporated without external heating. The residue was dissolved in tetrahydrofuran (15 ml) and the resulting solution treated with 2-mercaptopyrimidine (1.12 g) and potassium carbonate (3.00 g). The mixture was stirred at room temperature and evaporated and the residue partitioned between ethyl acetate and water. The layers were separated and the organic layer washed with water, dried over sodium sulphate and evaporated. The residue was purified by chromatography on silica (15 g) using toluene plus 0-100% ethyl acetate as eluant. Appropriate fractions were combined and evaporated and the residue triturated with diethyl ether. The resulting solid was collected, washed with ether and dried to give the title compound (121 mg), m.p. 151-153°C. Found: C,57.05; H,4.57; N,9.37; $C_{21}H_{20}ClN_3O_4S$ requires: C,56.56; H,4.49; N,9.43%.

## EXAMPLES 2-9

The following compounds were prepared by the method described in Example 1 by reacting 3,5-bis(methoxycarbonyl)-4-(2-chlorophenyl)-2,6-dimethyl-1,4-dihydropyridine with pyridinium bromide perbromide and then treating a solution of the resulting crude 3,5-bis(methoxycarbonyl)-2-bromomethyl-4-(2-chlorophenyl)-6-methyl-1,4-dihydropyridine in tetrahydrofuran with the appropriate thiol in the presence of potassium carbonate.

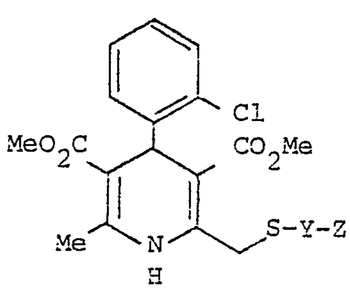

| Example No. | −Y−Z | m.p. (°C) | Analysis % (Theoretical in brackets) C | H | N |
|---|---|---|---|---|---|
| 2 | 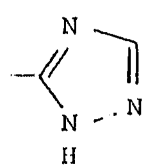 | 141−143 | 52.37<br>(52.47 | 4.51<br>4.37 | 12.52<br>12.89) |
| 3 | 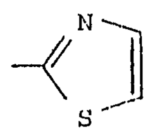 | 117−118 | 53.67<br>(53.27 | 4.32<br>4.22 | 6.30<br>6.21) |
| 4 | 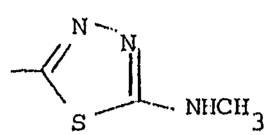 | 160−162 | 47.96<br>(47.95 | 4.28<br>4.59 | 11.92 ‡<br>11.19) |
| 5 | 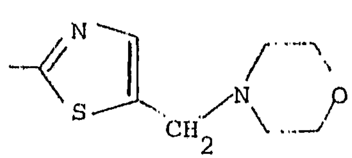 | 134−136 | 54.41<br>(54.59 | 5.18<br>5.09 | 7.51<br>7.64) |

| Example No. | -Y-Z | m.p. (°C) | Analysis % (Theoretical in brackets) C | H | N |
|---|---|---|---|---|---|
| 6 | 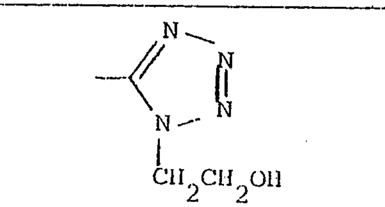 | 66-68 | 50.15<br>(50.05 | 4.84<br>4.59 | 14.20<br>14.60) |
| 7 | 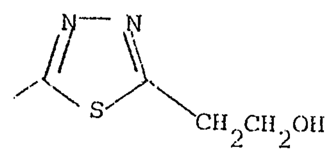 | 130-134 | 51.18<br>(50.85 | 4.49<br>4.47 | 8.12<br>8.47) |
| 8 | 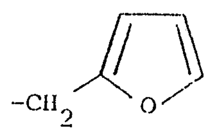 | 104-105 | 58.85<br>(58.98 | 5.00<br>4.91 | 3.33<br>3.13) |
| 9 | 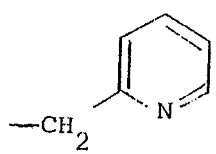 | 122-124 | 59.77<br>(60.19 | 4.94<br>5.02 | 5.99<br>6.10) |

‡ Characterised as a hydrate.

EXAMPLE 10

5-{[3.5-Bis(methoxycarbonyl)-4-(2.3-dichlorophenyl)-6-methyl-1.4-dihydropyrid-2-yl]methylthiomethyl}tetrazole

A solution of 2-{[3.5-bis(methoxycarbonyl)-4-(2,3-dichlorophenyl)-6-methyl-1,4-dihydropyrid-2-yl]methylthio}-acetonitrile (1.87 g) and tri-n-butyltin azide (1.45 g) in dioxane (30 ml) was heated under reflux for 17 hours and evaporated. The residue was taken up in diethyl ether (120 ml) and hydrogen chloride bubbled vigorously through the solution for 4o minutes. The mixture was stirred at room temperature for 16 hours and the resulting solid collected. washed with diethyl ether and dried to give the title compound (1.50 g). m.p 95-100 C. Found: C.46.73: H.4.08: N.14.15: $C_{19}H_{19}Cl_2N_5O_4S$ requires: C.47.11; H.3.95; N.14.46%.

EXAMPLES 11 and 12

5-{[3.5-Bis(methoxycarbonyl)-4-(2.3-dichlorophenyl)-6-methyl-1.4-dihydropyrid-2-yl]methylthiomethyl}-1-(2-hydroxyethyl)-tetrazole and
5-{[3,5-bis(methoxycarbonyl)-4-(2,3-dichlorophenyl)-6-methyl-1,4-dihydropyrid-2-yl]methylthiomethyl}-2-(2-hydroxyethyl) tetrazole

A mixture of 5-{[3,5-bis(methoxycarbonyl)-4-(2.3-dichlorophenyl)-6-methyl-1,4-dihydropyrid-2-yl]methylthiomethyl}-tetrazole (0.56 g), 2-bromoethanol (1.40 g) and potassium carbonate (0.70 g) in acetonitrile (20 ml) was heated under reflux for 1.5 hours and evaporated. The residue was partitioned between ethyl acetate and water and the layers separated. The organic layer was washed three times with water. dried over magnesium sulphate and evaporated. The residue was separated into two components by chromatography on silica (15 g) using dichloromethane plus 0-30% ethyl acetate followed by dichloromethane plus 30% ethyl acetate plus 1% methanol as eluant. In each case the appropriate fractions were combined and evaporated and the residual oil triturated with diethyl ether. The resulting solid was collected, washed with diethyl ether and dried to give each of the title compounds:-

(i) The less polar 2-isomer (95 mg) had m.p. 50-60°C. Found: C,48.23; H, 4.47; N,13.54; $C_{21}H_{23}Cl_2N_5O_5S$ requires: C,47.73; H,4.39; N,13.25%.

(ii) The more polar 1-isomer was characterised as a hemihydrate (80 mg) and had m.p. 65-75°C. Found: C,46.95: H,4.40; N,12.79; $C_{21}H_{23}Cl_2N_5O_5S0.5\ H_2O$ requires: C,46.93; H,4.50; N,13.03%.

EXAMPLE 13

5-{[3,5-Bis(methoxycarbonyl)-4-(2,3-dichlorophenyl)-6-methyl-1,4-dihydropyrid-2-yl]methylthiomethyl}-2-(2-dimethylaminoethyl)tetrazole

A mixture of 5-{[3,5-bis(methoxycarbonyl)-4-(2,3-dichlorophenyl)-6-methyl-1,4-dihydropyrid-2-yl]methylthiomethyl}-tetrazole (0.90 g), 2-dimethylaminoethyl chloride hydrochloride (0.80 g) and potassium carbonate (1.35 g) in acetonitrile (30 ml) was heated under reflux for 8 hours and then evaporated. The residue was partitioned between ethyl acetate and water and the layers separated. The organic layer was washed twice with water. dried over magnesium sulphate and evaporated. The residue was purified by chromatography on silica (10 g) using dichloromethane plus 0-3% methanol as eluant. Appropriate fractions were combined and evaporated to give the title compound (110 mg), m.p. 50-60°C. Found: C,50.36; H,5.10; N,15.16; $C_{23}H_{28}Cl_2N_6O_4S$ requires: C,49.73; H,5.08; N,15.13%.

EXAMPLE 14

1-<2-{[4-(2-Chlorophenyl)-3-ethoxycarbonyl-5-methoxycarbonyl-6-methyl-1,4-dihydropyrid-2-yl]methylthio}ethyl>-2.4.5-trimethylimidazole

Acetaldehyde ammonia (0.46 g) was added to a stirred, ice-cooled solution of 2-(2-aminoethyl)thiomethyl--4-(2-chlorophenyl)-3-ethoxycarbonyl-5-methoxycarbonyl-6-methyl-1,4-dihydropyridine (0.52 g) in methanol (5 ml) and the mixture stirred at 0°C for one hour. A solution of diacetyl (0.42 g) in methanol (2 ml) was then added dropwise to the mixture over 15 minutes. The mixture was stirred at 0° C for one hour, stored at 0° C for 16 hours, treated with concentrated aqueous ammonia solution (8 ml) and then stored at 0° C for 24 hours. The solution was evaporated and the residue purified by chromatography on silica (10 g) using dichloromethane plus 0-3% methanol as eluant. Appropriate fractions were combined and evaporated to give the title compound as a hemihydrate (87 mg), m.p. 168-170°C. Found: C,59.20; H,6.24; N,7.57. $C_{26}H_{32}ClN_3O_4S.{}^1/_2\ H_2O$ requires: C,59.26; H.6.27; N,7.98%.

EXAMPLE 15

2-Amino-6-{[4-(2,3-dichlorophenyl)-3-ethoxycarbonyl-5-methoxycarbonyl-6-methyl-1,4-dihydropyrid-2-yl]methylthiomethyl}-4-pyrimidone

A mixture of ethyl 4-{[4-(2.3-dichlorophenyl)-3-ethoxycarbonyl-5-methoxycarbonyl-6-methyl-1,4-dihydropyrid-2-yl]methylthio} acetoacetate (1.63 g). 1.5-diazabicyclo-[4.3.0]non-5-ene (1.24 g) and guanidine hydrochloride (0.93 g) in ethanol (30 ml) was stirred at room temperature for 40 hours and then partitioned

between ethyl acetate and water. The organic layer was washed twice with water, dried over sodium sulphate and evaporated. The residue was purified by chromatography on silica (18 g) using dichloromethane plus 25% ethyl acetate plus 0-15% methanol as eluant. Appropriate fractions were combined and evaporated and the residue triturated with diethyl ether. The resulting solid was collected, washed with diethyl ether and dried to give the title compound (0.35 g), m.p. 135-140° C. Found: C,50.85; H,4.67; N,10.03. $C_{23}H_{24}Cl_2N_4O_5S$ requires: C,51.21; H,4.45; N,10.39%.

EXAMPLE 16

4-<2-{[4-(2,3-Dichlorophenyl)-3-ethoxycarbonyl-5-methoxycarbonyl-6-methyl-1,4-dihydropyrid-2-yl]methylthio}ethyl>-1,2,4-triazole

A solution of 2-(2-aminoethyl)thiomethyl-4-(2,3-dichlorophenyl)-3-ethoxycarbonyl-5-methoxycarbonyl-6-methyl-1,4- dihydropyridine (1.00 g), N,N-dimethylformamide azine (0.50 g) and para-toluenesulphonic acid monohydrate (0.15 g) in toluene (30 ml) was heated under reflux for 14 hours and then evaporated. The residue was partitioned between ethyl acetate and 5% aqueous sodium carbonate solution and the layers separated. The organic layer was washed twice with water, dried over magnesium sulphate and evaporated. The residue was purified by chromatography on silica (8 g) using dichloromethane plus 0-40% ethyl acetate as eluant. Appropriate fractions were combined and evaporated to give the title compound (100 mg), m.p. 80-85° C. Found: C,51.32; H,4.84; N,10.75. $C_{22}H_{24}Cl_2N_4O_4S$ requires: C,51.66; H,4.73; N,10.96%.

EXAMPLE 17

2-<2-{[4-(2,3-Dichlorophenyl)-3-ethoxycarbonyl-5-methoxycarbonyl-6-methyl-1,4-dihydropyrid-2-yl]methylthio}ethylamino>pyrazine

A mixture of 2-(2-aminoethyl)thiomethyl-4-(2,3-dichlorophenyl)-3-ethoxycarbonyl-5-methoxycarbonyl-6-methyl-1,4-dihydropyridine (1.00 g), 2-chloropyrazine-1-oxide (0.43 g), triethylamine (2 ml) and 4-dimethylaminopyridine (0.10 g) in ethanol (10 ml) was heated under reflux for 29 hours and then evaporated. The residue was partitioned between ethyl acetate and 2M hydrochloric acid and the layers separated. The ethyl acetate layer was washed with water, dried over magnesium sulphate and evaporated. The residue was purified by chromatography on silica (8 g) using dichloromethane plus 25% hexane followed by dichloromethane plus 0-1% methanol as eluant. Appropriate fractions were combined and evaporated to give 2-<2-{[4-(2,3-dichlorophenyl)-3-ethoxycarbonyl-5-methoxycarbonyl-6-methyl-1,4-dihydropyrid-2-yl]methylthio}ethylamino>pyrazine-1-oxide (0.84 g) as an essentially pure oil.

Sodium dithionite monohydrate (0.5 g) was added to a solution of the above oil in a mixture of ethanol (20 ml) and water (20 ml) and the mixture heated under reflux for a total of 2.5 hours. During this period 0.5 g portions of sodium dithionite monohydrate were added to the mixture until at total of 2.5 g had been added. The mixture was evaporated and the residue partitioned between ethyl acetate and water. The layers were separated and the ethyl acetate layer washed twice with water, dried over magnesium sulphate and evaporated. The residue was purified by chromatography on silica (5 g) using hexane plus 50-100% dichloromethane followed by dichloromethane plus 1% methanol as eluant. Appropriate fractions were combined and evaporated to give the title compound as a hemihydrate (0.33 g), m.p. 65-70° C. Found: C,52.42; H,4.70; N,9.89. $C_{24}H_{26}Cl_2N_4O_5S.0.5\ H_2O$ requires: C,52.75; H,4.95; N,10.26%.

EXAMPLE 18

4-<2-{[4-(2,3-Dichlorophenyl)-3-ethoxycarbonyl-5-methoxycarbonyl-6-methyl-1,4-dihydropyrid-2-yl]methylthio}ethylamino>-6-(4-morpholino)pyrimidine

A solution of 2-(2-aminoethyl)thiomethyl-4-(2,3-dichlorophenyl)-3-ethoxycarbonyl-5-methoxycarbonyl-6-methyl-1,4-dihydropyridine (0.92 g), 4,6-dichloropyrimidine (0.16 g) and triethylamine (1.0 ml) in ethanol (15 ml) was heated under reflux for 18 hours and then evaporated. The residue was partitioned between dichloromethane and 2M hydrochloric acid and the layers separated. The organic layer was washed with water, dried over sodium sulphate and evaporated. The residue was purified by chromatography on silica (10 g) using dichloromethane plus 35% hexane followed by dichloromethane plus 0-1% methanol as eluant. Appropriate fractions were combined and evaporated to give 6-chloro-4-<2-{[4-(2,3-dichlorophenyl)-3-ethoxycarbonyl-5-methoxycarbonyl-6- methyl-1,4-dihydropyrid-2-yl]methylthio}-ethylamino>pyrimidine (0.30 g) as an essentially pure oil.

A solution of the above oil in morpholine (15 ml) was warmed on a steam-bath for 22 hours and then quenched into excess ice-cold 2M hydrochloric acid and extracted into ethyl acetate. The ethyl acetate extract was washed with water, dried over sodium sulphate and evaporated. The residue was purified by chromatography on silica (10 g) using dichloromethane plus 50% hexane followed by dichloromethane plus 0-1% methanol as eluant. Appropriate fractions were combined and evaporated to give the title compound (77 mg) as a hydrate, m.p. 65-67° C. Found: C,52.70; H,5.62; N,10.45. $C_{28}H_{33}Cl_2N_5O_5S.H_2O$ requires: C,52.50; H,5.47; N 10.93%.

EXAMPLE 19

2-<2-{[4-(2.3-Dichlorophenyl)-3-ethoxycarbonyl-5-methoxycarbonyl-6-methyl-1,4-dihydropyrid-2-yl]methylthio}ethylamino>pyrimidine

A solution of 2-(2-aminoethyl)thiomethyl-4-(2,3-dichlorophenyl)-3-ethoxycarbonyl-5-methoxycarbonyl-6-methyl-1.4-dihydropyridine (0.43 g). 2-chloropyrimidine (0.13 g) and triethylamine (1.0 ml) in ethanol (15 ml) was heated under reflux for 16 hours and then evaporated. The residue was purified by chromatography on silica using dichloromethane plus 50% hexane followed by dichloromethane plus 0-1% methanol as eluant. Appropriate fractions were combined and evaporated to give the title compound (134 mg) as an oil which was characterised as a hemihydrate. Found: C.52.99: H.4.96: N,10.11. $C_{24}H_{26}Cl_2N_4O_4S.0.5\ H_2O$ requires: C,52.74; H.4.94: N.10.25%.

EXAMPLE 20

5-Amino-3-<2-{[4-(2,3-dichlorophenyl)-3-ethoxycarbonyl-5-methoxycarbonyl-6-methyl-1,4-dihydropyrid-2-yl]methylthio}-ethylamino>-1,2,4-triazole

A solution of 3-cyano-1-<2-{[4-(2,3-dichlorophenyl)-3-ethoxycarbonyl-5-methoxycarbonyl-6-methyl-1,4-dihydropyrid-2-yl]methylthio}ethyl>2-methylisothiourea (0.81 g) and hydrazine hydrate (160µl) in acetonitrile (40 ml) was heated under reflux for 12 hours and then evaporated. The residue was partitioned between ethyl acetate and water and the organic layer washed with water, dried over sodium sulphate and evaporated. The residue was purified by chromatography on silica (5 g) using dichloromethane plus 0-5% methanol as eluant. Appropriate fractions were combined and evaporated to give the title compound (0.17 g) as an oil. Found: C,49.17; H.5.17; N,14.69. $C_{22}H_{26}Cl_2N_6O_4S$ requires: C,48.80; H,4.84; N,15.52%.

Preparation 1

2-{[3,5-Bis(methoxycarbonyl)-4-(2,3-dichlorophenyl)-6-methyl-1,4-dihydropyrid-2-yl]methylthio}acetonitrile

A solution of trifluoroacetic anhydride (1.68 g) in dioxane (20 ml) was added dropwise over 10 minutes to a stirred solution of 2-{[3,5-bis(methoxycarbonyl)-4-(2,3-dichlorophenyl)-6-methyl-1,4-dihydropyrid-2-yl]methylthio}acetamide (2.75 g) and pyridine (1.26 g) in dioxane (80 ml). The mixture was stirred at room temperature for 16 hours and then evaporated. The residue was dissolved in ethyl acetate and the solution washed once with 1M hydrochloric acid and once with 5% aqueous sodium carbonate solution, dried over magnesium sulphate and evaporated to give the title compound (1.96 g), m.p. 60-70°C. Found: C,51.66; H.4.25; N,6.07. $C_{19}H_{18}Cl_2N_2O_4S$ requires: C,51.70; H,4.11; N,6.35%.

Preparation 2

2-{[3.5-Bis(methoxycarbonyl)-4-(2,3-dichlorophenyl)-6-methyl-1,4-dihydropyrid-2-yl]methylthio}acetamide

N.N'-Carbonyl diimidazole (3.2 g) was added in one portion to a stirred solution of 2-{[3,5-bis(methoxycarbonyl)-4-(2,3-dichlorophenyl)-6-methyl-1,4-dihydropyrid-2-yl]methylthio}acetic acid (8.0 g) in tetrahydrofuran (150 ml). The mixture was stirred at room temperature for 2.5 hours and then gaseous ammonia was bubbled through the stirred solution for 30 minutes. The mixture was stirred at room temperature for 16 hours and then evaporated. The residue was dissolved in ethyl acetate and the solution washed three times with water, dried over magnesium sulphate and evaporated. The residue was purified by chromatography on silica (20 g) using dichloromethane plus 0-5% methanol as eluant. Appropriate fractions were combined and evaporated to give the title compound (2.75 g) as a hydrate, m.p. 70-80°C. Found: C,47.74; H,4.25; N,5.71. $C_{19}H_{20}Cl_2N_2O_5S.H_2O$ requires: C.47.80; H,4.64: N,5.87%.

Preparation 3

2-{[3.5-Bis(methoxycarbonyl)-4-(2,3-dichlorophenyl)-6-methyl-1,4-dihydropyrid-2-yl]methylthio}acetic acid

Pyridinium bromide perbromide (10.6 g) was added portionwise over 10 minutes to a stirred, ice-cooled solution of 3,5-bis(methoxycarbonyl)-4-(2,3-dichlorophenyl)-2,6-dimethyl-1,4-dihydropyridine (9.8 g) and pyridine (4.0 ml) in dichloromethane. The mixture was stirred at 0°C for 1.5 hours, washed twice with ice-cold 0.3 M hydrochloric acid. dried over magnesium sulphate and evaporated without external heating. The residue was dissolved in a mixture of dioxane (100 ml) and N,N-dimethylformamide (100 ml) and the solution treated with thioglycolic acid (11.0 g) and potassium carbonate (33.0 g). The mixture was stirred at room temperature for 16 hours and then evaporated. The residue was partitioned between 1M hydrochloric acid and ethyl acetate and the layers separated. The organic layer was extracted five times into saturated aqueous sodium hydrogen carbonate solution and the combined aqueous extracts were evaporated to 150 ml, acidified with concentrated hydrochloric acid and extracted three times into dichloromethane. The combined organic extracts were dried over magnesium sulphate and evaporated to give the title compound (8.0 g) as an essentially pure oil which was used directly in Preparation 2 without further purification.

Preparation 4

2-(2-Aminoethyl)thiomethyl-4-(2-chlorophenyl)-3-ethoxycarbonyl-5-methoxycarbonyl-6-methyl-1,4-dihydropyridine

Sulphuryl chloride (1.08 g) was added dropwise to stirred, ice-cooled N,N-dimethylformamide (10 ml) over 10 minutes and the resulting solution was then added slowly to a stirred, ice-cooled solution of 4-(2-chlorophenyl)-3-ethoxycarbonyl-2-hydroxymethyl-5-methoxycarbonyl-6-methyl-1,4-dihydropyridine (1.45 g) and imidazole (1.36 g) in N,N-dimethylformamide (20 ml). The mixture was stirred at 0°C for 20 minutes and at room temperature for 2 hours and then evaporated. The residue was partitioned between ethyl acetate and 2M hydrochloric acid and the layers separated. The organic layer was washed once with 2M hydrochloric acid and once with water, dried over sodium sulphate and evaporated. The residue was dissolved in tetrahydrofuran (10 ml) and the solution added to a stirred, ice-cooled mixture of 2-aminoethanethiol hydrochloride (1.13 g) and potassium carbonate (1.38 g) in tetrahydrofuran (20 ml). The mixture was stirred at 0°C for 2 hours and at room temperature for 8 days and then partitioned between ethyl acetate and water. The layers were separated and the organic layer washed with water, dried over sodium sulphate and evaporated. The residue was purified by chromatography on silica (14 g) using dichloromethane plus 0-25% ethyl acetate followed by dichloromethane plus 25% ethyl acetate plus 0-15% methanol as eluant. Appropriate fractions were combined and evaporated and the residue triturated with diethyl ether. The resulting solid was collected, washed with diethyl ether and dried to give the title compound (0.78 g), m.p. 116-119°C. Found: C,56.69; H,6.18; N,6.44. $C_{20}H_{25}ClN_2O_4S$ requires: C,56.54; H,5.89; N,6.60%.

Preparation 5

2-(2-Aminoethyl)thiomethyl-4-(2,3-dichlorophenyl)-3-ethoxycarbonyl-5-methoxycarbonyl-6-methyl-1,4-dihydropyridine was prepared by the method described in Preparation 4 using 4-(2,3-dichlorophenyl)-3-ethoxycarbonyl-2-hydroxymethyl-5-methoxycarbonyl-6-methyl-1,4-dihydropyridine instead of 4-(2-chlorophenyl)-3-ethoxycarbonyl-2-hydroxymethyl-5-methoxycarbonyl-6-methyl-1,4-dihydropyridine as the starting material. The product was obtained as an essentially pure oil which was used directly in Examples 16, 17, 18 and 19 and Preparation 12 without further purification.

Preparation 6

4-(2-Chlorophenyl)-3-ethoxycarbonyl-2-hydroxymethyl-5-methoxycarbonyl-6-methyl-1,4-dihydropyridine

N,N'-Carbonyl diimidazole (1.78 g) was added in one portion to a stirred, ice-cooled solution of 2-carboxy-4-(2-chlorophenyl)-3-ethoxycarbonyl-5-methoxycarbonyl-6-methyl-1,4- dihydropyridine (3.80 g) in tetrahydrofuran (60 ml) and the mixture stirred at 0°C for 2 hours and at room temperature for 2 hours. The mixture was then cooled to 0°C and treated portionwise over 10 minutes with lithium aluminium hydride (0.46 g). The mixture was stirred at 0°C for 15 minutes and then quenched into ice-cold 2M hydrochloric acid and extracted into ethyl acetate. The ethyl acetate extract was washed with 10% aqueous sodium carbonate solution and water, dried over sodium sulphate and evaporated. The residue was dissolved in methanol (60 ml) and the solution cooled to 0°C and then treated in one portion with sodium borohydride (0.46 g). The mixture was stirred at 0°C for 5 minutes and then quenched into 2M hydrochloric acid and extracted into ethyl acetate. The ethyl acetate extract was washed twice with water, dried over sodium sulphate and evaporated. The residue was purified by chromatography on silica (50 g) using dichloromethane plus 0-3% methanol as eluant. Appropriate fractions were combined and evaporated and the residue crystallised from diethyl ether to give the title compound (1.50 g), m.p. 124-125°C. Found: C,59.02; H,5.60; N,4.03. $C_{18}H_{20}ClNO_5$ requires: C,59.09; H,5.47; N,3.83%.

Preparation 7

4-(2,3-Dichlorophenyl)-3-ethoxycarbonyl-2-hydroxymethyl-5-methoxycarbonyl-6-methyl-1,4-dihydropyridine was prepared by the method described in Preparation 6 using 2-carboxy-4-(2,3-dichlorophenyl)-3-ethoxycarbonyl-5-methoxycarbonyl-6-methyl-1,4-dihydropyridine instead of 2-carboxy-4-(2-chlorophenyl)-3-ethoxycarbonyl-5-methoxycarbonyl-6-methyl-1,4-dihydropyridine as the starting material. The product had m.p. 130-131 C. Found: C,53.55; H,4.86; N,3.46. $C_{18}H_{19}Cl_2NO_5$ requires: C,54.00; H,4.75; N,3.50%.

Preparation 8

2-Carboxy-4-(2-chlorophenyl)-3-ethoxycarbonyl-5-methoxycarbonyl-6-methyl-1,4-dihydropyridine

A solution of 2-chlorobenzaldehyde (70.25 g), diethyl 2-oxosuccinate (90.4 g) and methyl 3-aminocrotonate (57 5 g) in methanol (800 ml) was heated under reflux for 6 hours and then evaporated. The residue was dissolved in dioxane (200 ml) and the solution treated with 5M aqueous sodium hydroxide solution (200 ml). The mixture was stirred at room temperature for 16 hours, evaporated to a volume of 200 ml, washed three times with ethyl acetate, acidified with 5M hydrochloric acid and extracted into ethyl acetate. The ethyl acetate extracts were washed with water, dried over sodium sulphate and evaporated. The residue was crystallised from diethyl ether to give the title compound (46 g), m.p. 154-156°C. Found: C,56.91; H,4.80; N,3.55. $C_{18}H_{18}ClNO_6$ requires: C,56.92; H,4.74; N,3.69%.

Preparation 9

2-Carboxy-4-(2.3-dichlorophenyl)-3-ethoxycarbonyl-5-methoxycarbonyl-6-methyl-1,4-dihydropyridine was prepared by the method described in Preparation 8 using 2,3-dichlorobenzaldehyde instead of 2-chlorobenzaldehyde. The product had m.p. 160-162° C. Found: C,51.72: H.4.19: N,3.43. $C_{18}H_{17}Cl_2NO_6$ requires: C,52.17; H,4.10; N,3.38.

Preparation 10

Ethyl 4-{[4-(2,3-dichlorophenyl)-3-ethoxycarbonyl-5-methoxycarbonyl-6-methyl-1,4-dihydropyrid-2-yl]methylthio}acetoacetate

N.N'-Carbonyl diimidazole (1.00 g) was added in one portion to a stirred solution of 2-{[4-(2.3-dichlorophenyl)-3-ethoxycarbonyl-5-methoxycarbonyl-6-methyl-1,4-dihydropyrid-2-yl]methylthio}acetic acid (2.80 g) in dichloromethane (30 ml) and the mixture stirred at room temperature for 1.5 hours and then added to a solution fo 2,2-dimethyl-1,3-dioxane-4,6-dione (0.87 g) and pyridine (0.60 ml) in dichloromethane (20 ml). The mixture was stirred at room temperature for 4 hours, washed twice with 2M hydrochloric acid and twice with water, dried over magnesium sulphate and evaporated. The residue was dissolved in ethanol (40 ml) and the solution heated under reflux for 8 hours and evaporated. The residue was purified by chromatography on silica (26 g) using toluene plus 0-100% dichloromethane followed by dichloromethane plus 1-5% ethyl acetate as eluant. Appropriate fractions were combined and evaporated to give the title compound (1.63 g) as an essentially pure oil which was used in Example 15 without further purification.

Preparation 11

2-{[4-(2,3-Dichlorophenyl)-3-ethoxycarbonyl-5-methoxycarbonyl-6-methyl-1,4-dihydropyrid-2-yl]methylthio}acetic acid

Sulphuryl chloride (3.24 g) was added dropwise over 10 minutes to stirred, ice-cooled N,N-dimethylformamide (20 ml) and the resulting solution was added to a stirred, ice-cooled solution of 4-(2,3-dichlorophenyl)-3-ethoxycarbonyl-2-hydroxymethyl-5-methoxycarbonyl-6-methyl-1,4-dihydropyridine (4.80 g) and imidazole (4.08 g) in N,N-dimethylformamide (60 ml). The mixture was stirred at 0°C for 15 minutes and at room temperature for 1.5 hours and then evaporated. The residue was partitioned between ethyl acetate and 1M hydrochloric acid and the layers separated. The organic layer was washed with water, dried over magnesium sulphate and evaporated. The residue was dissolved in tetrahydrofuran (20 ml) and the solution added to a stirred solution of methyl thioglycolate (1.20 ml) in tetrahydrofuran (60 ml) containing potassium carbonate (3.2 g). The mixture was stirred at room temperature for 26 hours and then evaporated. The residue was partitioned between ethyl acetate and water and the layers separated. The organic layer was washed with water, dried over sodium sulphate and evaporated. The residue was purified by chromatography on silica (40 g) using toluene plus 0-10% ethyl acetate as eluant. Appropriate fractions were combined and evaporated to give methyl 2- [4-(2,3-dichlorophenyl)-3-ethoxycarbonyl-5-methoxycarbonyl-6-methyl-1,4-dihydropyrid-2-yl]methylthio acetate (2.75 g) as an essentially pure oil.

2M Aqueous sodium hydroxide solution (100 ml) was added to a solution of the above oil (2.75 g) in dioxane (100 ml) and the mixture stirred at room temperature for 2.5 hours, acidified with 2M hydrochloric acid and extracted into ethyl acetate. The ethyl acetate extracts were washed twice with water, dried over sodium sulphate and evaporated to give the title compound (2.5 g) as an essentially pure oil which was used directly in Preparation 10 without further purification.

Preparation 12

3-Cyano-1-<2-{[4-(2,3-dichlorophenyl)-3-ethoxycarbonyl-5-methoxycarbonyl-6-methyl-1,4-dihydropyrid-2-yl]methylthio} ethyl>-2-methylisothiourea

A solution of 2-(2-aminoethyl)thiomethyl-4-(2,3-dichlorophenyl)-3-ethoxycarbonyl-5-methoxycarbonyl-6-methyl-1.4-dihydropyridine (1.00 g) and dimethyl N-cyanoimidodithiocarbonate (0.30 g) in ethanol (30 ml) was heated under reflux for 6 hours and then evaporated. The residue was purified by chromatography on silica (5 g) using hexane plus 50% dichloromethane followed by dichloromethane plus 0-1% methanol as eluant. Appropriate fractions were combined and evaporated to give the title compound (0.81 g) as an essentially pure oil which was used directly in Example 20 without further purification.

**Claims**

1. A compound having the formula:-

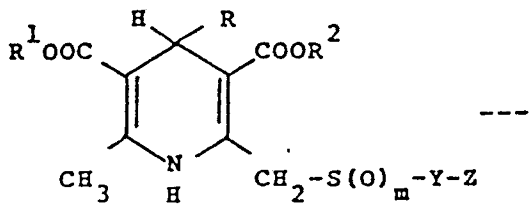

--- (I)

and pharmaceutically acceptable salts thereof, where R is an aryl or heteroaryl group;
$R^1$ and $R^2$ are each independently $C_1$-$C_4$ alkyl optionally substituted by $C_3$-$C_7$ cycloalkyl, aryl, $CF_3$, $C_2$-$C_4$ alkanoyl, halo, hydroxy, cyano, $C_1$-$C_4$ alkoxy, $C_2$-$C_4$ alkanoyloxy or $NR^3R^4$ where $R^3$ and $R^4$ are each independently H, $C_1$-$C_4$ alkyl, aryl-($C_1$-$C_4$ alkyl) or together with the nitrogen atom to which they are attached represent a pyrrolidinyl, piperidino, morpholino, piperazinyl, 4-($C_1$-$C_4$ alkyl)piperazine-1-yl or 4-formylpiperazin-1-yl group;
m is 0, 1 or 2;
Y is an optional alkylene linking group of from 0 to 4 carbon atoms which may be branched;
and Z is Het, -O-Het or -NH-Het where Het is an optionally substituted heterocyclic group.

2. A compound according to claim 1 wherein R is 2-chlorophenyl or 2,3-dichlorophenyl.

3. A compound according to claim 1 or claim 2 wherein $R^1$ is $CH_3$ and $R^2$ is $CH_3$ or $C_2H_5$, and m is 0.

4. A compound according to any one of claims 1 to 3 wherein Z is Het, -O-Het or -NH-Het and Het is a 5 or 6 membered heterocyclic group which may contain one or more N, S or O heteroatoms and which may be saturated, unsaturated or partially unsaturated and which may optionally be fused to a further 5 or 6 membered heterocyclic or carbocyclic ring and which may optionally be substituted by up to three substituents selected from $C_1$-$C_4$ alkyl, $C_1$-$C_4$ alkoxy, $C_2$-$C_5$ alkanoyl, halo, oxo, CN, $-(CH_2)_nCO_2H$, $-(CH_2)_nCO_2(C_1$-$C_4$ alkyl$)$, $-(CH_2)_nNR^3R^4$, $-(CH_2)_nCONR^3R^4$, $-(CH_2)_nNHCONR^3R^4$, $-(CH_2)_nNHCOCF_3$, $-(CH_2)_nNHCO(C_1$-$C_4$ alkyl$)$, $-(CH_2)_nNHSO_2(C_1$-$C_4$ alkyl$)$, $-(CH_2)_nNHSO_2$ aryl, $-(CH_2)_nNHSO_2NR^3R^4$, $-(CH_2)_nNHSO_2Het^1$, $-(CH_2)_nHet^1$, $-(CH_2)_nNHHet^1$, $-NH(CH_2)_nHet^1$, $-O-(CH_2)_nHet^1$, $-(CH_2)_nOH$, $-(CH_2)_n$aryl, -Oaryl, $-NH(CH_2)_pNR^3R^4$, $-C(C{=}X)NHR^5$, $-C({=}NCN)R^6$, $-C({=}NSO_2R^7)NH(C_1$-$C_4$ alkyl$)$, $-C({=}NH)NHR^8$, $-C({=}NCO(C_1$-$C_4$ alkyl$))NHCO(C_1$-$C_4$ alkyl$)$ and $-C({=}NR^9)SCH_3$;
wherein n is 0, 1, 2, 3 or 4; p is 2, 3 or 4;
$R^3$ and $R^4$ are as previously defined in claim 1;
$R^5$ is H, $C_1$-$C_4$ alkyl, $C_3$-$C_6$ cycloalkyl, $-CO_2(C_1$-$C_4$ alkyl$)$, $-CH_2CO_2(C_1$-$C_4$ alkyl$)$, aryl, $-SO_2$aryl or Het!;
X is O or S;
$R^6$ is $NR^3R^4$ or $-NH(CH_2)_2N(C_1$-$C_4$ alkyl$)_2$;
$R^7$ is $C_1$-$C_4$ alkyl or aryl;
$R^8$ is $-CONH(C_1$-$C_4$ alkyl$)$ or $-CO_2(C_1$-$C_4$ alkyl$)$;
$R^9$ is CN, $-SO_2(C_1$-$C_4$ alkyl$)$ or $-SO_2$aryl;
and $Het^1$ is a 5 or 6 membered nitrogen, oxygen, or sulphur containing heterocyclic group which may be saturated or unsaturated and which may optionally include a further one or two nitrogen atoms in the ring and which may optionally be benzofused or substituted with for example, halo, $C_1$-$C_4$ alkyl, $C_1$-$C_4$ alkoxy, trifluoroacetyl, hydroxy, oxo, acetamido, arylamino, $CONR^3R^4$, $NR^3R^4$ or $SO_2NR^3R^4$ groups where $R^3$ and $R^4$ are as previously defined in claim 1.

5. A compound as claimed in any one of claims 1 to 3 wherein Y is absent or is $CH_2$ or $(CH_2)_2$; Z is Het and is seleted from pyridyl, pyrimidinyl, pyrazolyl, furyl, imidazolyl, triazolyl, tetrazolyl, thiazolyl, thiadiazolyl and oxadiazolyl: each being optionally substituted by one or more substituents selected from $C_1$-$C_4$ alkoxy, carbamoyl, carbamoylmethyl, oxo, hydroxy, amino, methylamino, dimethylamino, dimethylaminoethyl, hydroxethyl, morpholinyl, and morpholinomethyl.

6. A compound as claimed in any one of claims 1 to 3 wherein Y is $(CH_2)_2$; Z is -NH-Het and Het is selected from 1,2,4-triazol-3-yl, 1,2,4-oxadiazol-3-yl, 1,2,5-thiadiazol-3-yl, pyrimidin-2-yl, pyrimidin-4-yl, imidazol-2-yl, 1,3,5-triazin-2-yl, pyrid-2-yl, thiazol-2-yl or pyrazin-2-yl; each being optionally substituted by one or more substituents selected from $C_1$-$C_4$ alkyl, $C_1$-$C_4$ alkoxy, halo, hydroxy, oxo, cyano, 3-methylureido, phenyl, phenoxy, pyridyl, acetyl, carbamoyl, methylcarbamoyl, ( $C_1$-$C_4$ alkoxy)carbonyl, $-NR^3R^4$, and $-SO_2NR^3R^4$ where $R^3$ and $R^4$ are as previously defined.

7. A compound as claimed in claim 1 wherein R is 2-chlorophenyl or 2,3-dichlorophenyl; $R^1$ is $CH_3$; $R^2$ is $CH_3$ or $C_2H_5$; m is O; Y is absent or is $CH_2$ or $(CH_2)_2$; Z is Het or NH Het and Het is a pyrimidinyl; 1,2 4-triazolyl, thiazolyl, thiadiazolyl, tetrazolyl, furyl, pyridyl, imidazolyl or pyrazinyl group; each being optionally substituted by one or more methyl, methoxy, oxo, amino, methylamino, dimethylaminoethyl, morpholino, morpholinomethyl or hydroxyethyl groups.

8. A compound as claimed in claim 7 wherein R is 2-chlorophenyl; $R^1$ and $R^2$ are both $CH_3$; Y is $CH_2$ and Z is 2-furyl or 2-pyridyl.

9. A pharmaceutical composition comprising a compound of the formula (I) as claimed in any one of the preceding claims, or a pharmaceutically acceptable salt thereof, together with a pharmaceutically

acceptable diluent or carrier.

10. A compound of the formula (I) as claimed in any one of claims 1 to 8, or a pharmaceutically acceptable salt thereof, for use in medicine including use in the prevention or treatment of cardiac conditions, or use as an antihypertensive, in man.

CLAIMS FOR THE FOLLOWING CONTRACTING STATES: AT ES GR

1. A process for preparing a compound of the formula:

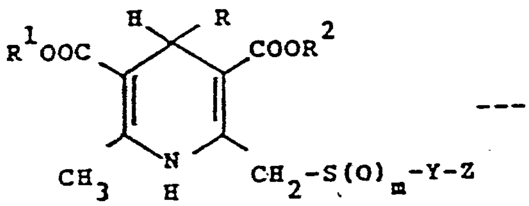

--- (I)

and pharmaceutically acceptable salts thereof,
where R is an aryl or heteroaryl group;
$R^1$ and $R^2$ are each independently $C_1$-$C_4$ alkyl optionally substituted by $C_3$-$C_7$ cycloalkyl, aryl, $CF_3$, $C_2$-$C_4$ alkanoyl, halo, hydroxy, cyano, $C_1$-$C_4$ alkoxy, $C_2$-$C_4$ alkanoyloxy or $NR^3R^4$ where $R^3$ and $R^4$ are each independently H, $C_1$-$C_4$ alkyl, aryl-($C_1$-$C_4$ alkyl) or together with the nitrogen atom to which they are attached represent a pyrrolidinyl, piperidino, morpholino, piperazinyl, 4-($C_1$-$C_4$ alkyl)piperazine-1-yl or 4-formylpiperazin-1-yl group;
m is 0, 1 or 2;
Y is an optional alkylene linking group of from 0 to 4 carbon atoms which may be branched;
and Z is Het, -O-Het or -NH-Het where Het is an optionally substituted heterocyclic group,
characterised by:
(a) reacting a compound of the formula:

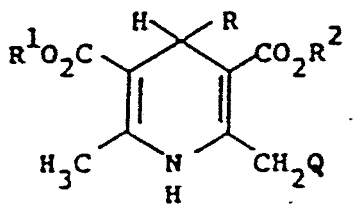

--- (II)

wherein R, $R^1$ and $R^2$ are as previously defined and Q is a good leaving group, with a thiol of the formula:
HS-Y-Het --- (III)
wherein Y and Het are as previously defined, to give compounds of the formula (I) wherein Z is Het and m is O: or
(b) reacting a compound of the formula:

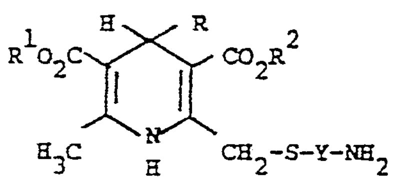

--- (IV)

wherein R, $R^1$ and $R^2$ are as previously defined and Y is an alkylene linking group of from 2 to 4 carbon atoms having at least 2 carbon atoms between S and $NH_2$; with a compound of the formula:
Het-Q --- (V)
wherein Q is a good leaving group to give compounds of the formula (I) wherein Z is -NH-Het and m is O; or
(c) reacting a compound of the formula:

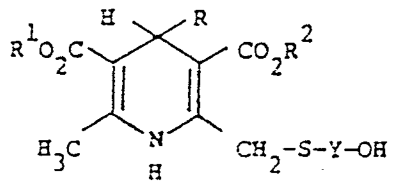

--- (VI)

wherein R, $R^1$ and $R^2$ are as previously defined and Y is an alkylene linking group of from 2 to 4 carbon atoms having at least 2 carbon atoms between S and O; with a strong base to form the anion, followed by addition of a compound of the formula (V), to give compounds of the formula (I) wherein Z is -O-Het and m is O: or

(d) reacting a compound of the formula:

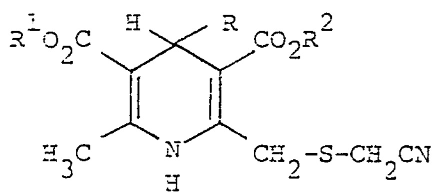

wherein R, $R^1$, and $R^2$ are as previously defined, with tri-n-butyltin azide, to yield the compound of formula (I) wherein Y is $CH_2$, Z is tetrazolyl and m is O; or

(e) reacting a compound of the formula (IV) with acetaldehyde ammonia and diacetyl, to give componds of formula (I) wherein m is O and Z is 2,4,5-trimethylimidazol-1-yl; or with N,N-dimethylformamide azine to give the compounds of the formula (I) wherein m is O and Z is 1,2,4-triazol-4-yl; or

(f) reacting a compound of the formula:

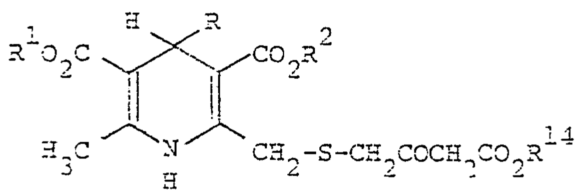

wherein R, $R^1$ and $R^2$ are as previously defined and $R^{14}$ is $C_1$-$C_4$ alkyl; with guanidine to yield the compounds of formula (I) wherein Y is $CH_2$, Z is 2-amino-4-pyrimidon-6-yl and m is O;

(g) reacting a compound of the formula

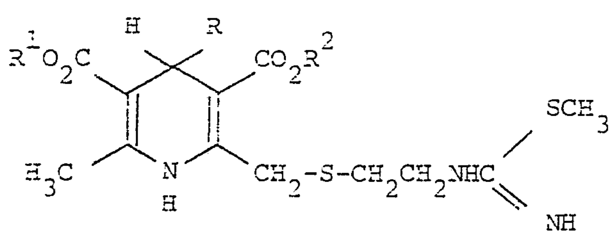

wherein R, $R^1$ and $R^2$ are as previously defined. with hydrazine to yield the compounds of formula (I) wherein Y is $(CH_2)_2$. Z is NH-Het wherein Het is 5-amino-1,2,4-triazol-3-yl and m is O;

and in each case optionally using a conventional chemical transformation reaction to introduce one or further substituent groups into the Het group; and optionally oxidising to give the compounds wherein m is 1 or 2 and optionally forming a pharmaceutically acceptable salt of the product.

2. A process as claimed in claim 1 wherein R is 2-chlorophenyl or 2,3-dichlorophenyl.

3. A process as claimed in claim 1 or claim 2 wherein $R^1$ is $CH_3$ and $R^2$ is $CH_3$ or $C_2H_5$ and the optional oxidation step is omitted.

4. A process as claimed in claim 1 wherein R is 2-chlorophenyl or 2,3-dichlorophenyl; $R^1$ is $CH_3$; $R^2$ is $CH_3$ or $C_2H_5$. m is O: Y is absent or is $CH_2$ or $(CH_2)_2$; Z is Het or NH Het and Het is a pyrimidinyl,

1,2,4-triazolyl, thiazolyl, thiadiazolyl, tetrazolyl, furyl, pyridyl, imidazolyl or pyrazinyl group; each being optionally substituted by one or more methyl, methoxy, oxo, amino, methylamino, dimethylaminoethyl, morpholino, morpholinomethyl or hydroxethyl groups.

5. A process as claimed in claim 1 for preparing a compound of the formula (I) wherein R is 2-chlorophenyl or 2,3-dichlorophenyl; $R^1$ is $CH_3$: $R^2$ is $CH_3$ or $C_2H_5$; m is O; Y is absent or is $CH_2$ or $(CH_2)_2$ and Z is Het where Het is pyrimidinyl, 1,2,4-triazolyl, thiazolyl, thiadiazolyl, tetrazolyl, furyl or pyridyl, each being optionally substituted by one or more methyl, methoxy, oxo, amino, methylamino, dimethylaminoethyl, morpholino, morpholinoethyl or hydroxyethyl groups, characterised by reacting a compound of the formula II, wherein R, $R^1$ and $R^2$ are as previously defined and Q is halo, paratoluenesulphonyloxy, methanesulphonyloxy, trifluoromethanesulphonyloxy or 1-imidazolylsulphonyloxy, with a compound of the formula (III) wherein Y and Het are as previously defined.

6. A process as claimed in claim 5 wherein Q is bromo and the reaction is performed in tetrahydrofuran at room temperature in the presence of potassium carbonate.

7. A process as claimed in claim 1 for preparing a compound of formula (I) wherein R is 2-chlorophenyl or 2,3-dichlorophenyl; $R^1$ is $CH_3$; $R^2$ is $CH_3$ or $C_2H_5$; m is O, Y is $(CH_2)_2$ and Z is -NH-Het where Het is pyrazinyl, pyrimidinyl or 1,2,4-triazolyl each being optionally substituted by one or more methyl, methoxy, halo, oxo, amino, methylamino, dimethylamino or morpholino groups characterised by reacting a compound of the formula (IV) wherein R, $R^1$, $R^2$ and Y are as previously defined with a compound of the formula (V) wherein Q is $NHNO_2$, $C_1$-$C_4$ alkylthio, $C_1$-$C_4$ alkoxy or halo and Het is as previously defined.

8. A process as claimed in claim 7 wherein Q is chloro and the reaction is performed by heating under reflux in ethanol in the presence of triethylamine.